(19) Europäisches Patentamt
European Patent Office
Office européen des brevets

(11) **EP 3 041 559 B1**

(12) **EUROPEAN PATENT SPECIFICATION**

(45) Date of publication and mention
of the grant of the patent:
**08.08.2018  Bulletin 2018/32**

(51) Int Cl.:
*A61M 16/12* *(2006.01)*    *A61M 16/06* *(2006.01)*
*A61M 16/10* *(2006.01)*    *A61M 16/20* *(2006.01)*
*A61M 16/04* *(2006.01)*

(21) Application number: **14740118.6**

(22) Date of filing: **27.06.2014**

(86) International application number:
**PCT/US2014/044494**

(87) International publication number:
**WO 2014/210417 (31.12.2014 Gazette 2014/53)**

(54) **BREATHING ASSISTANCE APPARATUS WITH NASAL CANNULA ASSEMBLY COMMUNICATING WITH A DEFORMABLE RESERVOIR**

HILFSGERÄT FÜR DIE BEATMUNG MIT NASENKANÜLENANORDNUNG ZUR KOMMUNIKATION MIT EINEM VERFORMBAREN BEHÄLTER

DISPOSITIF D'ASSISTANCE RESPIRATOIRE AVEC ENSEMBLE CANULE NASALE COMMUNICANT AVEC UN RÉSERVOIR DÉFORMABLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.06.2013   US 201313930357**
**28.06.2013   US 201313930460**
**28.06.2013   US 201313930407**
**28.06.2013   US 201313930435**
**28.06.2013   US 201313930503**
**28.06.2013   US 201313930545**

(43) Date of publication of application:
**13.07.2016   Bulletin 2016/28**

(73) Proprietor: **L'Air Liquide Société Anonyme pour l'Etude et l'Exploitation des Procédés Georges Claude 75007 Paris (FR)**

(72) Inventor: **MARTIN, Andrew Edmonton, Alberta T6J 5W9 (CA)**

(74) Representative: **Pittis, Olivier L'Air Liquide, S.A. Direction de la Propriété Intellectuelle 75, Quai d'Orsay 75321 Paris Cedex 07 (FR)**

(56) References cited:
**EP-A1- 0 010 880      WO-A1-2005/099801**
**WO-A1-2010/076711      GB-A- 2 493 520**
**US-A- 3 513 844      US-A- 6 089 229**
**US-A1- 2008 041 393      US-B1- 6 668 828**

## Description

### Background of the Invention

*Field of the Invention*

The invention concerns a breathing assistance apparatus comprising a nasal cannula assembly adapted to deliver gases to a patient for NO gas therapy.

### Description of the State of the Art

NO/nitrogen gas mixtures are commonly used for treating vasoconstrictions of the lung and pulmonary hypertension in adults and infants.

For instance, EP-A-1516639 discloses a gaseous mixture consisting of NO and an inert gas, preferably nitrogen, used for the production of an inhalable medicament for treating persistent pulmonary hypertension of the newborn.

Before being inhaled by the patient, the $NO/N_2$ mixture is generally diluted in an oxygen-containing gas, such as air or a $O_2/N_2$ mixture, comprising at least 21 vol.% of oxygen.

Typically, NO is present in the final mixture in an amount of several (1-800, most often 5- 80) ppm in volume.

However, as NO is rapidly oxidized into $NO_2$ in the presence of oxygen, it is important to avoid long residence times in gas administration apparatus between the point of mixing of the $NO/N_2$ mixture with the oxygen containing gas and inhalation by the patient, in order to keep the concentration of $NO_2$ in said inhalable medicament at less than 5 ppm, ideally less than 1 ppm, in the inhaled gas mixtures because $NO_2$ is a highly toxic gas.

NO gas mixtures are delivered by modified ventilation devices or special modules added to standard ventilators. Such devices are well known and taught, for instance, by US Patent Nos. 5,558,083; 5,873,359; 5,732,693; and 6,051,241.

Current NO delivery systems are designed for use with ventilators or other breathing gas delivery devices in a hospital or transport setting. Systems to deliver NO to ambulatory patients are in development. The majority of delivery devices are pulsed, sequential, or proportional delivery systems that sense the start of patient inhalation and use one or more electronically controlled valves or switches to deliver a sequenced flow of NO to the patient interface, for example an endotracheal tube, a facemask, or a nasal cannula.

For example, US Patent No. 6,089,229 discloses a device comprising sensing means for sensing the initiation of an inhalation of a patient and a delivery means responsive to the sensing means.

Further, US Patent No. 6,142,147 teaches an apparatus with a pressure sensor and a valve controller which is responsive to the pressure sensor, and which selectively connects a first port to a second port, said second port being connected to a source of NO gas, when a negative pressure event is sensed. Here the negative pressure event would be caused by a patient's inhalation so that again a means of sensing the patient's inhalation is used.

Furthermore, US Patent No. 6,581,599 deals with a method of delivering NO that includes detecting the onset of inspiration.

If adapted for NO delivery to ambulatory patients, such systems suffer from the requirements of a source of electrical power and the need for electromechanical parts to sense and administer sequenced pulses of NO, both of which increase the size of the system, and limit its portability. In addition, due to inevitable lags in timing between detection of the start of patient inhalation and operation of dosing valves, these systems risk delivering their pulses too late in the inhalation, such that a significant fraction of NO is exhaled.

However, there is sufficient evidence to suggest that long term NO therapy may be beneficial for some therapeutic indications, e.g. in treating pulmonary arterial hypertension. For these long term therapies, alternative delivery systems are needed for ambulatory patients. This is comparable to the need for devices for outpatient and in-home oxygen therapy.

For this purpose, a delivery system convenient for use by ambulatory patients, requiring a minimum of electromechanical parts, is required so that they can follow their NO treatment after they have left the hospital setting.

One common patient interface for home oxygen delivery is a standard form nasal cannula. Nasal cannulas are well known and widely used to deliver supplemental oxygen to patients suffering from a wide variety of respiratory and cardiovascular diseases. Generally, one end of an oxygen supply tubing is connected to a source of oxygen, and the other end of the tubing splits into two branches that meet to form a loop, where a set of two nasal prongs are positioned on that loop. The nasal prongs are inserted into a patient's nares, and a constant or time-pulsed flow of oxygen regulated by the source is sent through the tubing and the two branches of the loop so as to exit through the nasal prongs into the patient's nares. During inspiration, the patient inhales oxygen from the prongs together with entrained room air that is drawn through the space between the nasal prongs and the walls of the patient's nares. During exhalation, the patient exhales through the space between the nasal prongs and the walls of the patient's nares, and in the case of a constant oxygen supply flow, oxygen continues to exit into the patient's nares, such that much of this oxygen is carried with the expiratory flow into the surrounding room air. Pulsed oxygen delivery devices attempt to conserve oxygen by sensing the patient's breathing cycle, and then delivering a short-duration flow or pulse of oxygen through a nasal cannula only during inhalation, so as to avoid losing oxygen to the room air during exhalation.

As nasal cannulas are standard in the delivery of supplemental oxygen, many variants exist. For example, United States Patent 4,535,767 to Tiep et al. describes an oxygen delivery apparatus consisting of a reservoir cannula, a version of which is available as a commercial product called the Oxymizer from Chad Therapeutics, as described, for example by Dumont and Tiep (Using a reservoir nasal cannula in acute care; Crit Care Nurse 2002;22:41-46). This reservoir cannula includes a chamber in fluid communication with both the oxygen supply line and nasal prongs. The chamber is enclosed in part by a flexible diaphragm that collapses upon inhalation so as to empty its contents through the nasal prongs while at the same time blocking flow from the oxygen supply line to the chamber. The flexible diaphragm then expands during exhalation to fill the chamber with exhaled gas while re-establishing flow from the oxygen supply line into the chamber, such that oxygen from the supply line mixes with and displaces the exhaled gas through the nasal prongs. This type of reservoir cannula has found utility in supplying supplemental oxygen to patients, but is ill-suited for supplying patients with NO/nitrogen gas mixtures in place of oxygen. First, reservoir cannulas as previously described contain means to connect to only a single source of gas; however because commercial NO/nitrogen gas mixtures contain no oxygen, patients may require an additional source of supplemental oxygen. Second, even if air entrained from the room during inhalation provides sufficient oxygen to meet a patient's demand, it is not acceptable that oxygen-containing gas exhaled by the patient mix with NO-containing gas supplied to the chamber. It is well known that NO and oxygen react over time to produce $NO_2$, which is toxic even at relatively low concentrations (e.g. above 5 ppm short term or even 1 ppm for long term), and as such it is well accepted that the residence time during which NO is brought into contact with oxygen should be minimized when supplying these gases to a patient. Finally, the Oxymizer cannula delivers 20 mL of oxygen to the patient each breath. For commonly supplied concentrations of medical NO/nitrogen gas mixutres (e.g. containing 800ppm NO in balance nitrogen) this delivered volume risks exposing the patient to too high a concentration of NO and too low a concentration of oxygen, especially for younger patients with tidal volumes less than ~200 ml, or for adult patients exhibiting rapid, shallow breathing.

Another nasal cannula variant that exists is commonly referred to as a dual-lumen nasal cannula. For example TeleFlex Hudson RCI Dual Lumen Cannulas are commercially available. These cannulas connect through tubing to a source of oxygen and to a pressure sensing instrument, both of which are in fluid communication with a pair of nasal prongs, the cross section of each prong being split into two sections (or lumen) by a wall, with one section in fluid communication with the source of oxygen, and the other section in fluid communication with the pressure sensing instrument. While it is possible that one could conceive of connecting a source of NO-containing gas in place of the pressure sensing instrument in order to supply both NO and oxygen simultaneously through the dual-lumen cannula, no reservoir, chamber, or other mechanism is included to control the flow of gases. To provide a pulsed delivery of NO, one would need to rely on the systems described above that sense the start of patient inhalation and use one or more electronically controlled valves or switches to deliver a sequenced pulse of NO.

[0018] Further, US-A-6,089,229 discloses a respiratory therapy apparatus that delivers a predetermined pulsed dose of a therapeutic gas to a patient upon inhalation of the patient. The system has separate conduits for the supply of the therapeutic gas at a high concentration and for the supply of a diluent gas. The therapeutic gas conduit has a control valve that is accurately controlled to open and close so as to deliver a precise dose of the high concentration therapeutic gas. The diluent gas conduit has means to ensure there is sufficient flow of diluent gas to ensure the said high concentration therapy gas is diluted to a safe concentration. A third conduit receives the gases from both the first and second conduits so that the gases are mixed together and administered to the patient.

Furthermore, WO-A-2010/076711 teaches a respiration appliance system for supporting the airway of a subject. The flow of gas from the lungs of the subject during exhalation is leveraged to provide support to the airway. In particular, a body that encloses one or more external orifices of the subject provides a resistance differential between inhaled gas flows and exhaled gas flows that supports the subject's airway.

**Brief Summary of the Invention**

A main goal of the invention is to provide a delivery system convenient for use by ambulatory patients, which allows nitric oxide (NO) to be efficiently administered over extended time periods, i.e. hours, days, weeks, through nasal prongs in a manner that minimizes delivery into the anatomical dead volume at the end of inhalation, and therefore also minimizes exhalation of NO. In so doing, the system must avoid bringing NO-containing gas into contact with oxygen-containing gas until just prior to delivery to the patient, so as to avoid or minimize production of toxic $NO_2$ gas through reaction of NO with oxygen.

Another goal is to provide a delivery system that, in contrast to pulsed delivery systems described in prior art, does not require a sensor to detect the onset of inspiration nor any processing unit (such as a PLC or programmable computer) or other electronics. The solution according to the present invention consists of a breathing assistance apparatus comprising:

    a) a source of NO-containing gas, and
    b) a nasal cannula assembly in fluid communication with said source of NO-containing gas, the nasal cannula assembly adapted to deliver gases to a patient, wherein the nasal cannula assembly comprises:

- a hollow body configured to be capable of acting as a gas conduct or a gas manifold and comprising an internal chamber defining a first compartment,
- the first compartment and a second compartment separated by a separation wall,
- a pair of nasal prongs in fluid communication with the first compartment,
- the first compartment comprising a first inlet forming a side gases entry in fluid communication with a gas transport conduct and configured to conduct a first gas into said first compartment,
- the second compartment having a fully inflated internal volume for a gas, the second compartment comprising,

    • a second inlet configured to conduct a second gas into said second compartment, said second gas being NO-containing gas from the source of NO-containing gas, and
    • a deformable wall forming a part of the boundary between the second compartment and the room atmosphere,

and wherein the separation wall comprises at least two flow restriction channels.

Depending on the embodiment, the breathing assistance apparatus according to the present invention can comprise one or several of the following features:

- the nasal cannula assembly further comprises a hollow body comprising an internal chamber comprising at least the first compartment.
- at least the first compartment is part of a hollow body forming a gas conduct or a manifold.
- said hollow body and said pair of nasal prongs are integrally molded from a soft plastics material.
- the prongs are detachable from said hollow body to allow different sized prongs to be placed on said hollow body to suit different sized patients.
- the flow restriction channels have reentrant apertures for limiting the return flow of gas.
- a pair of flow restriction channels is arranged in the separation wall, directly opposite the pair nasal prongs.
- the second compartment forms a deformable-wall reservoir comprising an internal volume for the gas, when fully inflated, of about 0.5 to 5 ml.
- said source of NO-containing contains NO and nitrogen.
- said source of NO-containing contains up to 3000 ppm in volume of NO in a balance of nitrogen.

It is also disclosed a method for treating pulmonary vasoconstriction in a patient, comprising:

    a) providing a breathing assistance apparatus according to the present invention, and
    b) providing a therapeutically-effective amount of a NO-containing gas to said patient through the nasal cannula assembly of said breathing assistance apparatus for inhalation.

Said method for treating pulmonary vasoconstriction can comprise one or several of the following features:

- the patient is an adult, an infant or a newborn.
- pulmonary vasoconstriction is associated with persistent pulmonary hypertension of the newborn.
- pulmonary vasoconstriction is associated with pulmonary arterial hypertension.
- the NO-containing gas is mixed with an oxygen-containing gas just before being inhaled by the patient.
- the NO-containing gas is a NO/nitrogen mixture.
- the NO-containing gas consists in a NO/nitrogen mixture containing up to 3000 ppm by volume of NO.
- the $O_2$-containing gas is air or an $O_2/N_2$ mixture containing at least 21vol.% of $O_2$.

In some embodiments, the breathing assistance apparatus according to the present invention can comprise the features defined by one or more of the following numbered sentences:

    3. The deformable wall of the second compartment of the nasal cannula assembly has a greater Compliance while filling than when the second compartment is full.
    4. The nasal prongs of the nasal cannula assembly include an external pillow element at an end.
    5. The pillow element of the nasal cannula assembly is made of silicone.
    6. The nasal cannula assembly further comprises an expiratory orifice , optionally comprises a one-way expiratory valve, between the first compartment defining an internal chamber and an external atmosphere.
    7. The nasal cannula assembly further comprises a resistive element between an internal volume of the first compartment and an air or oxygen supply, the resistive element configured to ensure a sufficient pressure drop upon the onset of inhalation to open at least one valve element.
    8. The source of NO-containing contains from 1 ppm to 5000 ppm in volume of NO in a balance of nitrogen.
    9. The prongs are detachable from said hollow body and selected from different sized prongs suitable for different sized patient nares.
    10. The at least two flow restriction channels connect the first compartment and second compartment and are rounded edged at the entrance from second compartment and have a reentrant aperture at the entrance from the first compartment.

11. The at least two flow restriction channels are two flow restriction channels arranged in the separation wall (6), directly opposite the pair nasal prongs.

12. The second compartment forms a deformable-wall reservoir comprising a fully inflated internal volume for the gas of about 0.5 to 5 ml.

13. The at least two flow restriction channels prevent a majority of flow of the second gas from the second compartment to the first compartment in a higher pressure state, during an exhalation phase, relative to the passage of gas during a reduced pressure state, during an inhalation phase, *optionally* further causing the deformable wall to deform and increase the volume of the second compartment by at least one cubic centimeter in volume or by at least 50% volume, or both, as compared to the second compartment volume at the end of the previous reduced pressure state inhalation phase.

14. The at least two flow restriction channels prevent > 70% of flow of the second gas from the second compartment to the first compartment in a higher pressure state, during an exhalation phase, relative to the passage of gas during a reduced pressure state, during an inhalation phase.

15. The at least two flow restriction channels prevent > 90% of flow of the second gas from the second compartment to the first compartment in a higher pressure state, during an exhalation phase, relative to the passage of gas during a reduced pressure state, during an inhalation phase.

16. The deformable wall of the second compartment has a greater Compliance while filling than when the second compartment is full.

17. The diameter of the at least two flow restriction channels is between 0.1 mm and 5 mm, and the at least two flow restriction channels comprise rounded edges at an entrance to the at least two flow restriction channels designed such that the ratio between a radius of curvature of the rounded edges and the diameters of the at least two flow restriction channels is greater than 0.02.

**Brief Description of the Several Views of the Drawings**

The present invention will be better understood thanks to the following description and explanation made in reference to the Figures, wherein:

- Figure 1 is a schematic of a nasal cannula assembly of a breathing assistance apparatus according to a first comparative example,
- Figure 2 is a schematic of a nasal cannula assembly of a breathing assistance apparatus according to a second comparative example having nasal nare pillows for securing the cannula to the nares,
- Figure 3 is a schematic of a nasal cannula assembly of a breathing assistance apparatus according to a third comparative example,
- Figure 4 is a schematic of a nasal cannula assembly of a breathing assistance apparatus according to a first embodiment of the present invention having nasal nare pillows for securing the cannula to the nares,
- Figures 5A-D show a schematic of a nasal cannula assembly of a breathing assistance apparatus according to a second embodiment of the present invention with gas flow illustrated in the inhalation and exhalation phases with supplemental Oxygen (5A-B) and without supplemental Oxygen (5C-D), and
- Figure 6 displays an estimated pattern of inhaled NO concentration that could be achieved using a nasal cannula assembly of a breathing assistance apparatus according to the present invention,
- Figure 7 shows a schematic of a working model for validating restriction flow channel (35) performance under simulated breathing conditions, with elements labeled as follows:

[40] - Lung Simulator (ASL 5000; Ingmar Medical)
[22] - 22mm straight connector with sampling port
[300] - Gas sampling line to NO analyzer (Siemens NOA 280i; GE)
[21] - 22mm T piece connector
[23] - 22mm straight connector
[24] - Step-down connector, 22mm to 4mm
[35a] - 4 mm flow restriction channel
[25] - 22mm straight connector with injection port
[90] - NO injection line
[26] - Breathing filter (ClearGuard II; Intersurgical),

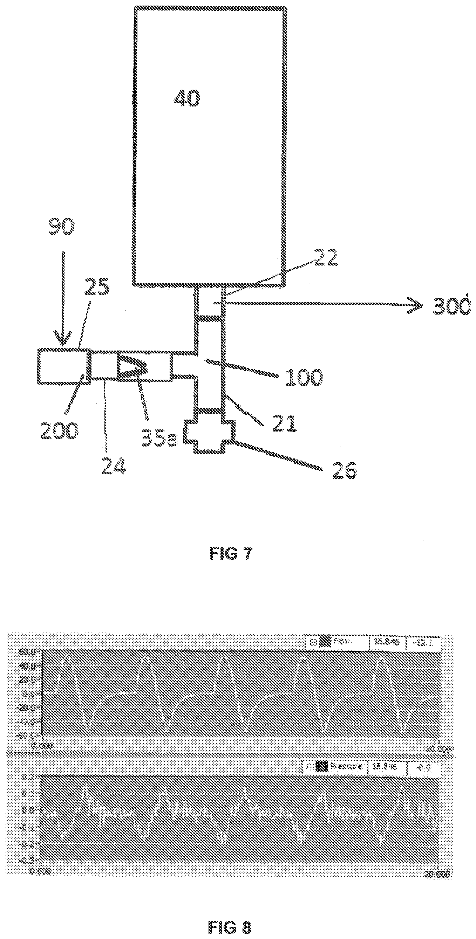

- Figure 8 shows results of an exemplary experiment using the testing apparatus of Figure 7: Flow (top) and pressure (bottom) versus time waveforms obtained from the lung simulator with no filter. The X-axis units are seconds, while the Y-axis units are L/min and cm H2O, respectively,

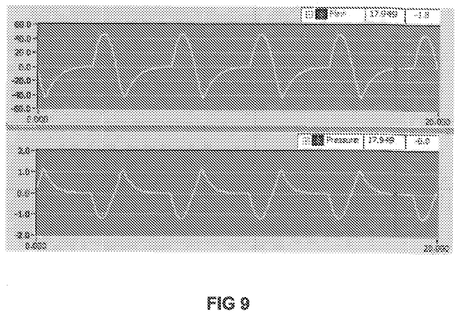

Figure 9 shows results of an exemplary experiment using the testing apparatus of Figure 7: Flow (top) and pressure (bottom) versus time waveforms obtained from the lung simulator with the filter in place. The X-axis units are seconds, while the Y-axis units are L/min and cm $H_2O$, respectively, and

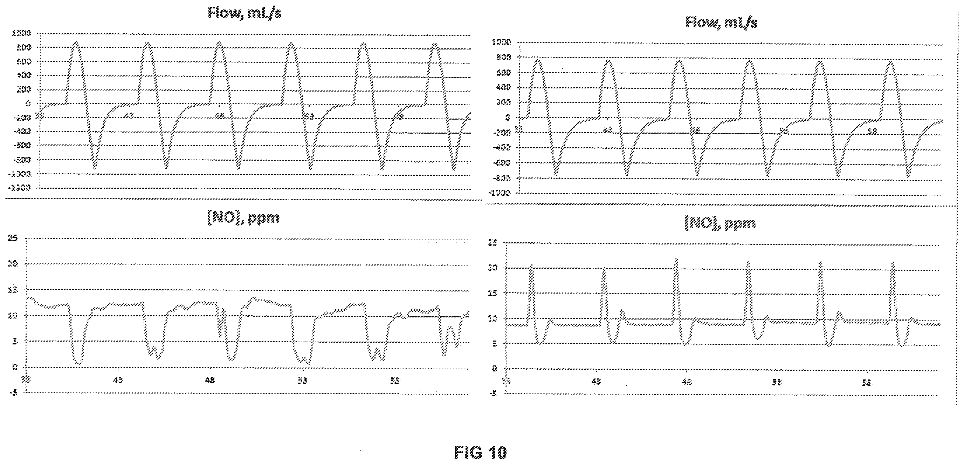

Figure 10 shows results of an exemplary experiment using the testing apparatus of Figure 7: *Top row:* flow versus time waveforms from the lung simulator obtained with no filter (left) and with the filter in place (right). *Bottom row:* NO concentration versus time waveform as sampled from the first compartment [1] with no filter (left) and with the filter in place (right).

## Detailed Description of the Invention

VALVED COMPARATIVE EXAMPLES

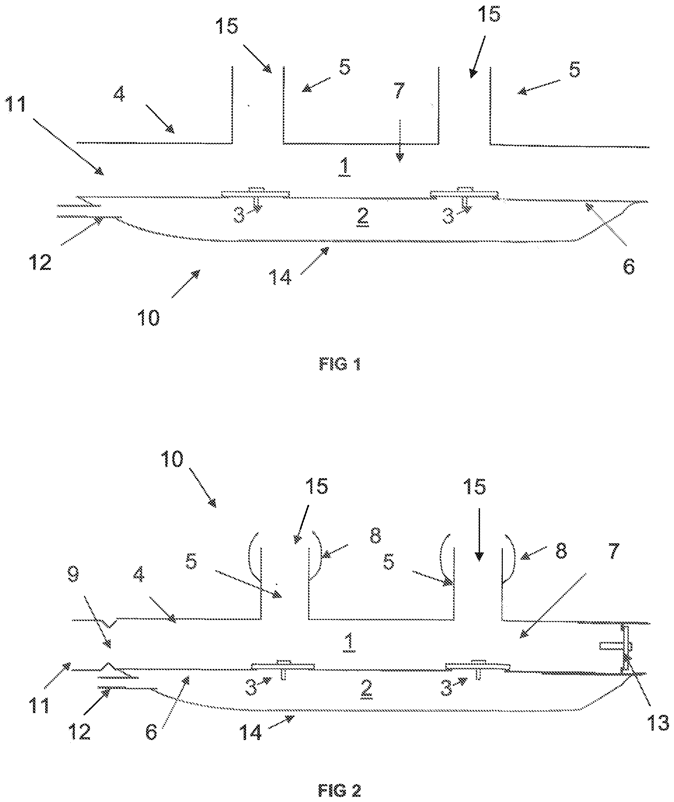

A schematic of the nasal cannula assembly of a breathing assistance apparatus according to a first comparative example is shown in Figure 1 (in cross section).

The nasal cannula assembly of the breathing assistance apparatus according to the first comparative example is a patient interface generally comprising a pair of nasal prongs 5 coupled indirectly to a deformable-wall 14 having a valved 3 reservoir 2 supplied with NO contained in nitrogen 12, e.g. at 225, 450, 800 or 1000 ppm in volume.

The pair of nasal prongs 5 is positioned on a hollow body 4, for example an air or oxygen conduit or manifold, comprising an internal hollow volume or chamber 7 thereby forming a first compartment 1 that receives the gases.

The nasal prongs 5 are small conduits or tubes adapted for insertion into the nares of a patient and through which passes the gas mixture that is subsequently inhaled by the patient. Each prong 5 comprises an outlet orifice 15 at its end.

The hollow body 4 can be made of a rigid light material, such as polymer or similar. Preferably, the hollow body 4 and the pair of nasal prong 5 are integrally molded from a soft plastics material. However, the prongs 5 can also be detachable from said hollow body 4 to allow different sized prongs to be placed on said hollow body 4 to suit different sized patients, such as adults and infants. The hollow body 4 is in turn in fluid communication with valved 3 reservoir 2 formed by the deformable-wall 14 and separation wall 6. In other words, the nasal cannula assembly of the present invention is split into two main inhaled gas compartments 1 and 2 that are separated each from the other by a separation wall 6.

The second compartment 2 forms a deformable reservoir 2 receiving the NO gas through an inlet 12. The deformable wall 14 of the reservoir or second compartment 2 should be made from a thin, flexible sheet of polymer material so that the reservoir readily inflates during exhalation when the valves 3 are closed, but collapses, at the start of inhalation, after the valves 3 open. In this manner, NO-containing gas is allowed to accumulate in the reservoir while the patient exhales, and then is released as a bolus at the start of inhalation as the reservoir collapses and its contents empty through the valves 3 into the first compartment 1. Throughout this cycle a constant flow of NO-containing gas may be maintained through the inlet 12.

The second compartment 2 fluidly communicates with the first compartment 1 through one or more fluid transfer elements such as the exemplary one-way valves 3 which may be for instance the two umbrella valves 3 shown in Figure 1. These fluid transfer elements are arranged in the separation wall 6. Preferably, as shown in Figure 1, two valves 3 are positioned along the conduit 7 forming the hollow main body 4, directly opposite the nasal prongs 5, i.e. each valve 3 is facing one nasal prong 5, so as to facilitate the gas circulation from the second compartment 2 to the first compartment 1 and subsequently to the nasal prongs 5.

The fluid transfer elements may be umbrella valves, duckbill valves, valve-like conduits or flow constrictions apertures designed to permit gas flow from the second compartment 2 to the first compartment 1, but to resist flow in the opposite direction from the first compartment 1 to the second compartment 2. In examples having valves 3, the valves preferably respond to any drop in ambient pressure (i.e. due to inhalation). Ideally, valve 3 is selected to have a "cracking pressure" of zero or at least as low as is mechanically feasible. Any inhalation flow through nare tubes 5 should preferably result in valve 3 opening.

In any case, the combination of valves 3 and deformable wall 14 of the embodiment depicted in Figure 1 should be responsive to increases and decreases in pressure that develop during exhalation and inhalation, respectively, so as to allow the deformable reservoir 2 to inflate with NO-containing gas during exhalation, and then empty to release this gas through the valves 3 into the first compartment 1 during inhalation.

Generally the deformable wall 14 needs to be of a thin, flexible material such that zero or near zero positive pressure above atmospheric develops in compartment 2 as it fills from the Nitric Oxide flow 12 during exhalation (so that the valves stay closed while the bag fills). Reservoir 2 thus should be designed preferably to have infinite or near infinite Compliance (where Compliance= deltaVolume/deltaPressure), while filling - and then drop to zero or near zero Compliance once full.

The first compartment 1 is supplied with an oxygen-containing gas through a first inlet port 11, whereas the second compartment 2 forming a NO-reservoir is supplied with a constant flow of NO-containing gas through a second inlet port 12.

During patient exhalation, the valves 3 close, so that the second compartment or reservoir 2 fills with NO containing gas, whereas, during patient inhalation, the valves 3 open so that NO-containing gas mixes with air and/or oxygen in the first compartment 1 as it is inhaled by the patient through the prongs 5.

The volume of the second compartment 2 is configured and sized so as to be small compared to the patient's inhaled tidal volume, so that the second compartment 2 quickly empties during the initial period of the inhalation phase to create a bolus of elevated NO concentration at the start of the inhalation.

Normally high concentrations of NO, e.g. 800 vol. ppm of NO in nitrogen, are delivered to the second compartment 2 from a source of NO/$N_2$, such as a gas cylinder with integrated pressure regulator and flow metering apparatus.

Patient safety is ensured by supplying only low flows of NO-containing gas. For example, to deliver to the patient an amount of NO equivalent to that delivered during continuous supply of gas containing 5 ppmv NO throughout the duration of a 500 ml tidal breath, about 3 ml of gas containing 800 ppmv NO should be supplied each breath.

During tidal breathing, the expiratory time of a typical adult will range from approximately 2 to 5 seconds. Therefore, supply flows on the order of 1 ml/s of NO containing gas are required. In operation, the NO flow rate may be adjusted based on visual inspection of the inflation/deflation of deformable wall **14** to ensure the appropriate flow rate for a specific patient's inhalation pattern.

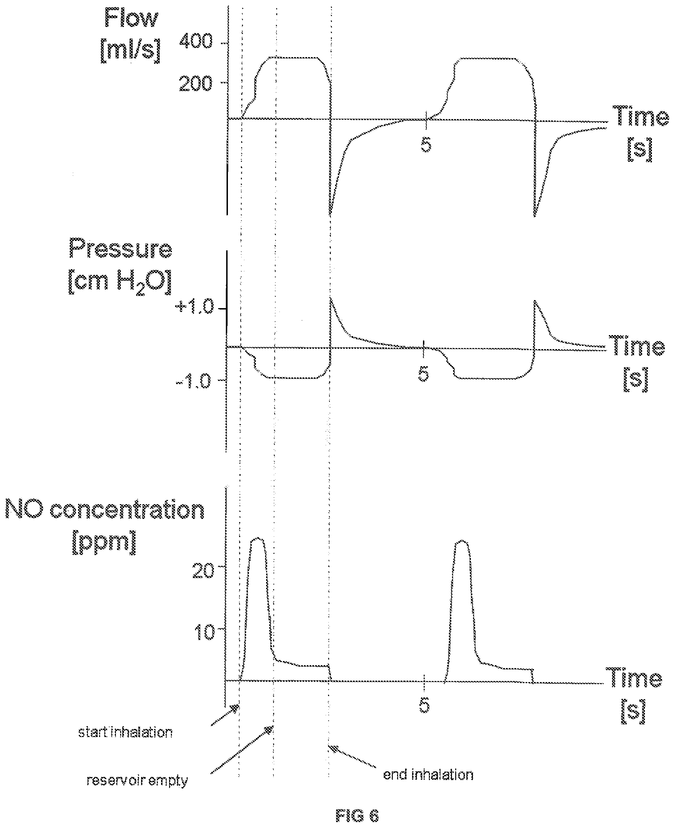

Figure 6 displays an estimated pattern of inhaled NO concentration that would be achieved using the first comparative example based on the numbers mentioned above.

More precisely, one can see on Fig. 6 the estimated tidal flow and inhaled NO concentration curves during a typical adult tidal breathing pattern using a nasal cannula assembly of a breathing assistance apparatus **10** according to the first comparative example supplied with 1 ml/sec flow of gas containing 800 ppmv of NO in $N_2$.

The breathing pattern is shown in the upper curve, with positive flow representing inhalation, and negative flow representing exhalation. The estimated NO concentration contained in the gas mixture delivered to the patient through the nasal prongs **5** during the inhalation phase of the breathing cycle is shown in the lower curve.

The NO concentration spikes at the start of inhalation as NO-containing gas is released from the second compartment **2** before rapidly decreasing once the second compartment empties.

Through the later stages of inhalation a low NO concentration is delivered as fresh NO-containing gas supplied through inlet **12** passes into the first chamber **1** and the nasal prongs **5**.

In contrast, throughout exhalation, flow of NO-containing gas from the second chamber to the first chamber is prevented by the valves **3**.

For some patients, it may be desirable to minimize gas leaks between the nasal prongs **5** and the patient's nares, e.g. to provide continuous positive airway pressure CPAP, Bi-level positive airway pressure (Bi-PAP), or other positive pressure support in combination with NO therapy, or to ensure the proper opening and closing function of the valves **3**.

In such circumstances, the nasal cannula assembly **10** may comprise additional elements as shown in Figure 2.

First, each of the nasal prongs **5** includes an external pillow element **8** at its ends, which is intended to more tightly secure the prongs **5** inside the patient's nares or nostrils. Said pillow elements **8** can be made of soft resilient material, such as silicone or similar.

Second, an additional one-way expiratory valve **13** is included between the first compartment **1** defining the internal chamber **7** and the room atmosphere.

Additionally, a resistive element **9** may optionally be placed between the internal volume **7** of the first compartment **1** and the air or oxygen supply conduit to ensure a sufficient pressure drop upon the onset of inhalation to open valves **3**.

RESTRICTION FLOW CHANNEL EMBODIMENTS AND COMPARATIVE EXAMPLES

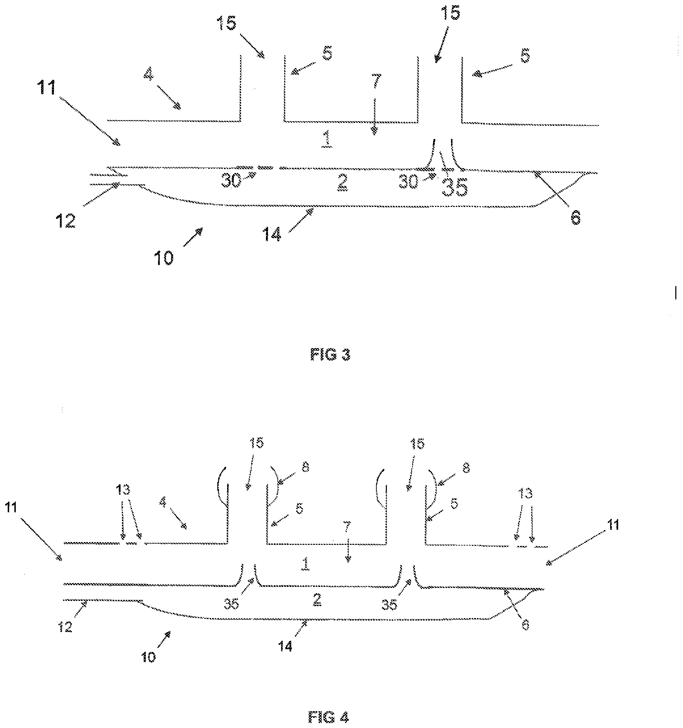

A schematic of the nasal cannula assembly of the breathing assistance apparatus according to a third comparative example is shown in Figure 3 (in cross section).

The nasal cannula assembly of the breathing assistance apparatus of the present invention is a patient interface generally comprising a pair of nasal prongs **5** coupled indirectly to a deformable-wall **14** having a reservoir **2** supplied with NO contained in nitrogen (**12**), e.g. at 225, 450, 800 or 1000 ppm in volume.

The pair of nasal prongs **5** is positioned on a hollow body **4**, for example an air or oxygen conduit or manifold, comprising an internal hollow volume or chamber **7** thereby forming a first compartment **1** that receives the gases.

The nasal prongs **5** are small conduits or tubes adapted for insertion into the nares of a patient and through which passes the gas mixture that is subsequently inhaled by the patient. Each prong **5** comprises an outlet orifice **15** at its end.

The hollow body **4** can be made of a rigid light material, such as polymer or similar. Preferably, the hollow body **4** and the pair of nasal prong **5** are integrally molded from a soft plastics material. However, the prongs **5** can also be detachable from said hollow body **4** to allow different sized prongs to be placed on said hollow body **4** to suit different sized patients, such as adults and infants. The hollow body **4** is in turn in fluid communication via fluid transfer elements **30**, such as flow restriction channel(s) **35**, with reservoir **2** formed by the deformable-wall **14** and separation wall **6**. In other words, the nasal cannula assembly of the present invention is split into two main inhaled gas compartments **1** and **2** that are separated each from the other by a separation wall **6**.

The second compartment **2** forms a deformable reservoir **2** receiving the NO gas through an inlet **12**. The deformable wall **14** of the reservoir or second compartment **2** should be made from a thin, flexible sheet of polymer material so that the reservoir readily inflates during exhalation, but collapses, at the start of inhalation. In this manner, NO-containing gas is allowed to accumulate in the reservoir while the patient exhales, and then is released as a bolus at the start of inhalation as the reservoir collapses and its contents empty through the fluid transfer elements **30**, such as flow restriction channel(s), into the first compartment 1. Throughout this cycle a constant flow of NO-containing gas may be maintained through the inlet **12**.

The second compartment 2 fluidly communicates with the first compartment 1 through one or more fluid transfer elements 30 as shown in Figure 3. These fluid transfer elements are arranged in the separation wall 6. Preferably, as shown in Figure 3, two fluid transfer elements 30 are positioned along the conduit 7 forming the hollow main body 4, directly opposite the nasal prongs 5, i.e. each fluid transfer element 30 is facing one nasal prong 5, so as to facilitate the gas circulation from the second compartment 2 to the first compartment 1 and subsequently to the nasal prongs 5.

The fluid transfer elements 30 are generally one or more flow restriction channel(s) 35 connecting first compartment 1 to second compartment 2, said one or more flow restriction channel(s) 35 being one flow restriction channel 35 in a breathing assistance apparatus according to a comparative example (Figure 3), and being at least two flow restriction channels 35 in a breathing assistance apparatus according to the present invention (Figure 4). The flow restriction channel(s) 35 should be adapted by dimension to limit the majority of flow of NO from second compartment 2 to first compartment 1 (i.e. ≥50% such as ≥60, ≥70, ≥80 or ≥90%) in the higher pressure state during an exhalation phase relative to the reduced pressure state during an inhalation phase. The flow rate differential between the two pressure conditions may be adjusted by selecting the appropriate flow restriction channel 35 dimensions (e.g. tubular size), a degree of baffling in the flow restriction channel 35, and/or any other suitable flow restriction elements (e.g. membranes, particle packing, constrictions, etc.). Independent of the specific geometric adaptation, the flow restriction element may be characterized by the equation:

$\Delta P = \frac{1}{2}\rho K V^2$, where $\Delta P$ indicates the pressure drop associated with flow of a fluid with density $\rho$ through the flow restriction element at a velocity $V$ representing the mean fluid velocity through the flow restriction element, as averaged, e .g., over the cross-section of the entrance to the element. The coefficient $K$ depends on the geometry and configuration of the flow restriction element, and may thus be used to characterize the flow restriction element, where a larger value of the coefficient $K$ is associated with a larger pressure drop through the flow restriction element for a given fluid density $\rho$ of a fluid traveling at a given mean velocity $V$. In other words, a larger value of the coefficient $K$ is associated with a lower mean flow velocity $V$ when a given pressure drop $\Delta P$ is imposed across the flow restriction element. Therefore a flow restriction element with larger coefficient $K$ will in general represent a larger barrier to flow through that element.

In light of this understanding, one adaptation of the flow restriction channel 35 connecting first compartment 1 to second compartment 2 is an orifice with dimension selected to produce a coefficient $K$ sufficiently large in value to limit flow from compartment 2 to compartment 1 through the flow restriction channel during the higher pressure state, where the higher pressure in compartment 1 is associated with a small pressure drop $\Delta P$ imposed across the orifice, but at the same time sufficiently small in value to permit flow from compartment 2 to compartment 1 through the flow restriction channel during the reduced pressure state, where the reduced pressure in compartment 1 is associated with a larger pressure drop $\Delta P$ imposed across the orifice. A circular orifice with diameter between around 0.1 mm and around 5 mm, and specifically between 0.5 mm and 2 mm serves as a reasonable solution for many patient breathing patterns. Orifices of different geometry (e.g. an oval, a square, or a slot) but of similar dimension are also reasonable solutions.

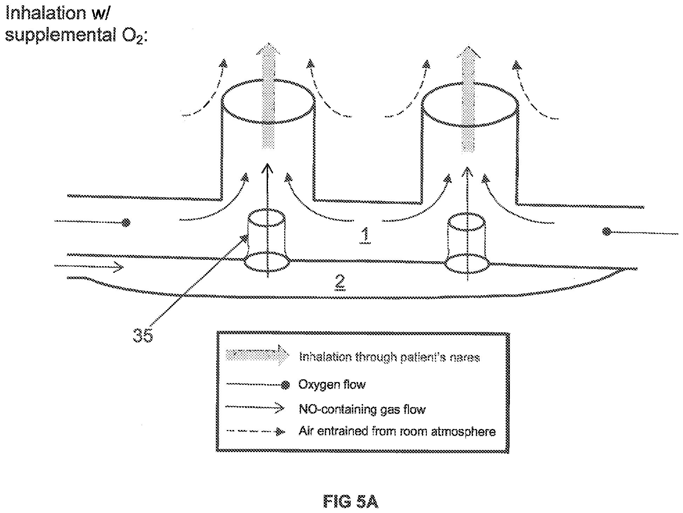

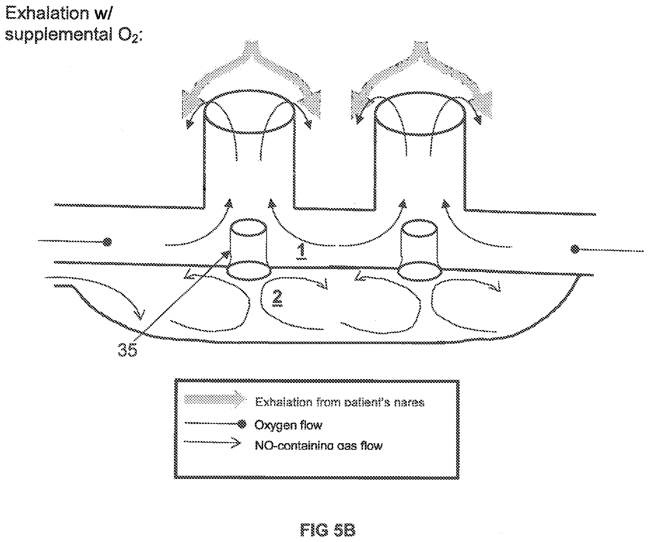

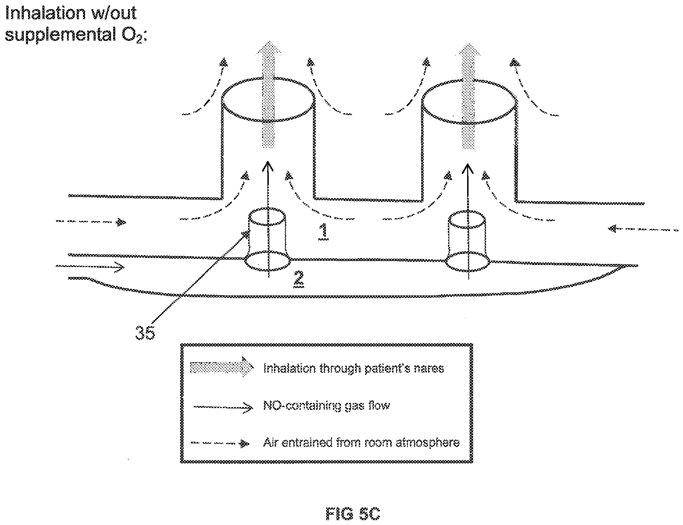

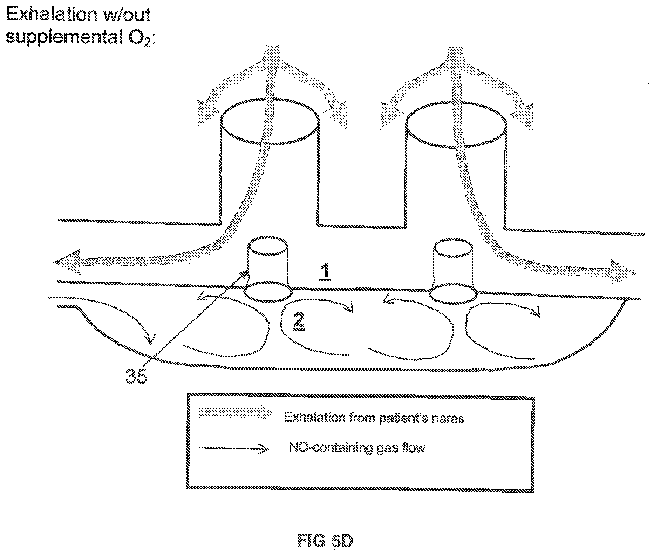

A second adaptation of the flow restriction channel 35 connecting first compartment 1 to second compartment 2 is a constriction channel (Figure 5A & 5B) designed such that the constriction channel has a rounded edge at the entrance from compartment 2 for flow in the direction from compartment 2 into compartment 1. Further, the constriction channel protrudes into compartment 1 so as to create a reentrant type entrance for flow in the reverse direction from compartment 1 into compartment 2. It is known that the coefficient $K$ defined above is considerably larger for a reentrant-type entrance than for a rounded-edged entrance, thus in the context of the flow restriction channel discussed here, use of a constriction channel as described above will provide little resistance to flow in the direction from compartment 2 into compartment 1 but will resist flow in the direction from compartment 1 to compartment 2. Accordingly, the constriction channel provides a further advantage for controlling the direction of flow between compartment 1 and compartment 2 in addition to performing the function of modulating flow from compartment 2 to compartment 1 between higher and reduced pressure states as described above for the flow restriction channel in general. The diameter of the constriction channel should generally be between around 0.1 mm and around 5 mm, and specifically between 0.5 mm and 2 mm. The rounded edges at the entrance to the constriction channel should be designed such that the ratio between the radius of curvature of the edge and the diameter of the constriction is greater than 0.02, preferably greater than 0.1; however, increasing this ratio beyond a value of about 0.15 provides little further benefit. For general guidance on restriction flow channel design, see Fox and McDonald, Introduction to Fluid Mechanics, Fifth edition, John Wiley & Sons, NY, 1998, Chapter 8, or Shaughnessy, Katz, and Schaffer, Introduction to Fluid Mechanics, Oxford University Press, NY, 2005, Chapter 13.

In any case, the combination of flow restriction channel(s) 35 and deformable wall 14 of the embodiment depicted in Figure 3 should be responsive to increases and decreases in pressure that develop during exhalation and inhalation, respectively, so as to allow the deformable reservoir 2 to inflate with NO-containing gas during exhalation, and then empty to release this gas through the flow restriction channel(s) **35** into the first compartment **1** during inhalation.

Generally the deformable wall **14** needs to be of a thin, flexible material such that zero or near zero positive pressure above atmospheric develops in compartment **2** as it fills from the Nitric Oxide flow **12** during exhalation (so that flow of gas through the restriction channel(s) **35** remains minimal during exhalation while the bag fills). Reservoir **2** thus should be designed preferably to have infinite or near infinite Compliance (where Compliance=deltaVolume/ deltaPressure), while filling - and then drop to zero or near zero Compliance once full.

The first compartment **1** is supplied with an oxygen-containing gas through a first inlet port **11**, whereas the second compartment **2** forming a NO-reservoir is supplied with a constant flow of NO-containing gas through a second inlet port **12**.

During patient exhalation, the second compartment or reservoir **2** fills with NO containing gas, whereas, during patient inhalation, NO-containing gas mixes with air and/or oxygen in the first compartment **1** as it is inhaled by the patient through the prongs **5**.

The volume of the second compartment **2** is configured and sized so as to be small compared to the patient's inhaled tidal volume, so that the second compartment **2** quickly empties during the initial period of the inhalation phase to create a bolus of elevated NO concentration at the start of the inhalation.

Normally high concentrations of NO, e.g. 800 vol. ppm of NO in nitrogen, are delivered to the second compartment **2** from a source of $NO/N_2$, such as a gas cylinder with integrated pressure regulator and flow metering apparatus.

Patient safety is ensured by supplying only low flows of NO-containing gas. For example, to deliver to the patient an amount of NO equivalent to that delivered during continuous supply of gas containing 5 ppmv NO throughout the duration of a 500 ml tidal breath, about 3 ml of gas containing 800 ppmv NO should be supplied each breath.

During tidal breathing, the expiratory time of a typical adult will range from approximately 2 to 5 seconds. Therefore, supply flows on the order of 1 ml/s of NO containing gas are required. In operation, the NO flow rate may be adjusted based on visual inspection of the inflation/deflation of deformable wall **14** to ensure the appropriate flow rate for a specific patient's inhalation pattern. Visual inspection of the inflation/deflation of the deformable wall **14** also provides feedback to the user to ensure proper function of the device, and may be used by a healthcare practitioner in fitting a patient with appropriately sized nasal prongs.

Figure 6 displays an estimated pattern of inhaled NO concentration that would be achieved using the present invention or the third comparative example based on the numbers mentioned above.

More precisely, one can see on Fig. 6 the estimated tidal flow, first compartment **1** pressure, and inhaled NO concentration curves during a typical adult tidal breathing pattern using a nasal cannula assembly **10** of the breathing assistance apparatus according to the present invention or to the third comparative example supplied with 1 ml/sec flow of gas containing 800 ppmv of NO in $N_2$.

The breathing pattern is shown in the upper curve, with positive flow representing inhalation, and negative flow representing exhalation. The variation of pressure within the first compartment **1** over the breathing cycle is shown in the middle curve. The estimated NO concentration contained in the gas mixture delivered to the patient through the nasal prongs **5** during the inhalation phase of the breathing cycle is shown in the bottom curve.

The NO concentration spikes at the start of inhalation as NO-containing gas is released from the second compartment **2** before rapidly decreasing once the second compartment empties.

Through the later stages of inhalation a low NO concentration is delivered as fresh NO-containing gas supplied through inlet **12** passes into the first chamber **1** and the nasal prongs **5**.

In contrast, throughout exhalation, flow of NO-containing gas from the second chamber to the first chamber is prevented or at least reduced by the flow restriction channel(s) **35**.

For some patients, it may be desirable to minimize gas leaks between the nasal prongs **5** and the patient's nares, e.g. to provide continuous positive airway pressure CPAP, Bi-level positive airway pressure (Bi-PAP), or other positive pressure support in combination with NO therapy, or to provide additional control over the higher and reduced pressure states achieved during the breathing cycle. In such circumstances, the nasal cannula assembly **10** may comprise additional elements as shown in Figure 4.

First, each of the nasal prongs **5** includes an external pillow element **8** at its ends, which is intended to more tightly secure the prongs **5** inside the patient's nares or nostrils. Said pillow elements **8** can be made of soft resilient material, such as silicone or similar.

Second, additional orifices or slots **13** are included between the first compartment **1** defining the internal chamber **7** and the room atmosphere, to allow entrainment of room air, e.g. during inhalation, and exhaust of gases to the room atmosphere, e.g. during exhalation. The number and dimensions of these orifices or slots **13** may be selected so as to achieve a desired range of higher and reduced pressure states during the breathing cycle.

*Working Example*

To demonstrate the restriction flow channels of the breathing assistance apparatus of the invention, a mechanical lung simulation device was used to produce a breathing pattern. The breathing pattern was set to that of an average adult human. Breathing patterns representing any patient could be used as the basis for validating a restriction flow channel design for a specific class of patients (e.g. pediatric breathing patterns). The exemplary experiment was performed using a single flow restriction channel [35a] positioned between a first compartment [100] and a second compartment [200]. A 22mm diameter straight connector [22] including a port for gas sampling via the gas sampling line [300] was positioned between a lung simulator (ASL 5000; Ingmar Medical) [40] and T piece [21]. A second arm of the T piece [21] was connected to a 22mm straight connector, which contained internally a 4 mm flow restriction channel [35a]. The third arm of the T piece [21] was either left open to the room atmosphere, or connected to a breathing filter (ClearGuard II; Intersurgical) [26], which in turn was open to the room atmosphere. The internal conduits of the straight connector with sampling port [22], the T piece [21], and the straight connector [23] formed the first compartment [100]. The flow restriction channel [35a] was connected through a step-down connector [24] to a 22mm diameter straight connector [25] which included a port for NO gas injection from the NO gas injection line [90]. The opposite end of the straight connector [25] was open to the room atmosphere. The internal conduits of the straight connector with injection port [25] and the step-down connector [24] formed a second compartment [200].

Flow into and out of the lung simulator and the pressure at the entrance to the lung simulator, representing the pressure throughout the first compartment [100], were recorded over time by the lung simulator. The concentration of NO in gas sampled via the gas sampling line [300] was monitored using a chemiluminescence NO analyzer (Siemens NOA 280i; GE). The lung simulator was programmed so as to deliver a 600 mL tidal volume breath at a frequency of 15 breaths/minute, with a sinusoidal inspiratory waveform and a passive, mono-exponential expiratory flow pattern, and an inspiratory time to expiratory time ratio of 2 to 3. A constant flow of 250 mL/min of 800ppm NO in balance nitrogen gas was delivered through the NO injection line [90]. Figures 8 and 9 display flow and pressure versus time waveforms over several breathing cycles for experiments performed without and with the filter [26] in place, respectively. The flow waveform is not appreciably changed between the two experiments, while the pressure waveforms differ. With no filter in place, figure 8, the pressure in the first compartment [100] oscillates between a minimum of ~-0.2 cm $H_2O$ during inhalation and a maximum of ~ 0.1 cm $H_2O$ during exhalation. With the filter [26] in place, figure 9, the small added resistance to air flow through the filter results in a reduced pressure state reaching -1.0 cm $H_2O$ during inhalation, and a higher pressure state reaching 1.0 cm $H_2O$ during exhalation.

Figure 10 again displays flow versus time waveforms (top row) over several breathing cycles for experiments performed without (top left) and with (top right) the filter [26] in place. The bottom row of Figure 10 displays NO concentrations versus time in gas sampled from the first compartment [100] for experiments performed without (bottom left) and with (bottom right) the filter [26] in place. With no filter in place, that is with pressure states in the first compartment [100] varying between -0.2 cm $H_2O$ during inhalation and 0.1 cm $H_2O$ during exhalation, the NO concentration in gas sampled from the first compartment [100] reached just over 10 ppm during periods of low flow (e.g. at end-expiration), where pressure in the first compartment [100] was near zero, and during inhalation, where pressure in the first compartment [100] was negative, but fell to near zero when expiratory flow rates were appreciable and the pressure in the first compartment [100] rose. With the filter [26] in place, such that the pressure in compartment [100] varied between a reduced pressure state reaching -1.0 cm $H_2O$ during inhalation, and a higher pressure state reaching 1.0 cm $H_2O$ during exhalation, the variation in delivered NO concentration was magnified: a bolus of gas containing NO concentration as high as 20 ppm passed through the first compartment [100] during inhalation, and the NO concentration fell to approximately 5 ppm during periods where expiratory flow was appreciable, such that the higher pressure state was achieved in the first compartment [100], before rising to 10 ppm through the remainder of the breathing cycle.

## Claims

1. A breathing assistance apparatus comprising:

   a) a source of NO-containing gas, and
   b) a nasal cannula assembly (10) in fluid communication with said source of NO-containing gas, the nasal cannula assembly (10) adapted to deliver gases to a patient, wherein the nasal cannula assembly (10) comprises:

      A) a hollow body (4) configured to be capable of acting as a gas conduct or a gas manifold and comprising an internal chamber (7) defining a first compartment (1),
      B) the first compartment (1) and a second compartment (2) separated by a separation wall (6),
      C) a pair of nasal prongs (5) in fluid communication with the first compartment (1),
      D) the first compartment (1) comprising a first inlet (11) forming a side gases entry in fluid communication with a gas transport conduct and configured to conduct a first gas into said first compartment (1),
      E) the second compartment (2) having a fully inflated internal volume for a gas, the second compartment (2) comprising,

. a second inlet (12) configured to conduct a second gas into said second compartment (2), said second gas being NO-containing gas from the source of NO-containing gas, and

. a deformable wall (14) forming a part of the boundary between the second compartment (2) and the room atmosphere,

and wherein the separation wall (6) comprises at least two flow restriction channels (35).

**2.** The breathing assistance apparatus according to claim 1, wherein said hollow body (4) and said pair of nasal prongs (5) are integrally molded from a soft plastics material.

**3.** The breathing assistance apparatus according to claim 1, wherein the prongs (5) are detachable from said hollow body (4) and selected from different sized prongs suitable for different sized patient nares.

**4.** The breathing assistance apparatus according to claim 1, wherein the at least two flow restriction channels (35) connect the first compartment (1) and second compartment (2) and are rounded edged at the entrance from second compartment (2) and have a reentrant aperture at the entrance from the first compartment (1).

**5.** The breathing assistance apparatus according to claim 1, wherein the at least two flow restriction channels (35) are two flow restriction channels (35) arranged in the separation wall (6), directly opposite the pair nasal prongs (5).

**6.** The breathing assistance apparatus according to claim 1 wherein the second compartment (2) forms a deformable-wall reservoir comprising a fully inflated internal volume for the gas of about 0.5 to 5 ml.

**7.** The breathing assistance apparatus according to claim 1, wherein the nasal cannula assembly does not comprise a sensor configured to detect an onset of patient inspiration.

**8.** The breathing assistance apparatus according to claim 1, wherein the nasal prongs (5) include an external pillow element (8) at an end.

**9.** The breathing assistance apparatus according to claim 8, wherein said pillow element (8) is made of silicone.

**10.** The breathing assistance apparatus according to claim 1, further comprising one or more orifices (13) between the first compartment (1) defining an internal chamber (7) and an external atmosphere.

**Patentansprüche**

**1.** Hilfsgerät zur Beatmung, umfassend:

a) eine Quelle für NO-haltiges Gas, und
b) eine Nasenkanülenanordnung (10) in Fluidverbindung mit der Quelle für NO-haltiges Gas, wobei die Nasenkanülenanordnung (10) zur Abgabe von Gasen an einen Patienten angepasst ist,

wobei die Nasenkanülenanordnung (10) umfasst:

A) einen Hohlkörper (4), ausgelegt, um zum Wirken als eine Gasleitung oder ein Gasverteiler befähigt zu sein und umfassend eine Innenkammer (7), die ein erstes Kompartiment (1) definiert,
B) das erste Kompartiment (1) und ein zweites Kompartiment (2), die durch eine Trennwand (6) getrennt sind,
C) ein Paar von Naseneinsätzen (5) in Fluidverbindung mit dem ersten Kompartiment (1),
D) das erste Kompartiment (1), das einen ersten Einlass (11) umfasst, der einen seitlichen Gaseingang in Fluidverbindung mit einer Gastransportleitung ausbildet und zum Leiten eines ersten Gases in das erste Kompartiment (1) ausgelegt ist,
E) das zweite Kompartiment (2), das ein vollständig aufgeblähtes Innenvolumen für ein Gas aufweist, wobei das zweite Kompartiment (2) umfasst

. einen zweiten Einlass (12), ausgelegt zum Leiten eines zweiten Gases in das zweite Kompartiment (2), wobei das zweite Gas NO-enthaltendes Gas aus der Quelle für NO-haltiges Gas ist, und
. eine verformbare Wand (14), die einen Teil der Grenze zwischen dem zweiten Kompartiment (2) und der Raumatmosphäre ausbildet,

und wobei die Trennwand (6) mindestens zwei Strömungseinengungskanäle (35) umfasst.

**2.** Hilfsgerät zur Beatmung nach Anspruch 1, wobei der Hohlkörper (4) und das Paar von Naseneinsätzen (5) einstückig aus einem weichen Kunststoffmaterial geformt sind.

**3.** Hilfsgerät zur Beatmung nach Anspruch 1, wobei die Einsätze (5) aus dem Hohlkörper (4) lösbar sind und aus unterschiedlich bemessenen Einsätzen ausgewählt sind, die für unterschiedlich bemessene Patientennasenlöcher geeignet sind.

**4.** Hilfsgerät zur Beatmung nach Anspruch 1, wobei die mindestens zwei Strömungseinengungskanäle (35) das erste Kompartiment (1) und zweite Kompartiment (2) verbinden und am Eingang vom zweiten Kompartiment (2) rundgerändert sind und einen einspringenden Durchbruch am Eingang vom ersten Kompartiment (1) aufweisen.

**5.** Hilfsgerät zur Beatmung nach Anspruch 1, wobei die mindestens zwei Strömungseinengungskanäle (35) zwei in der Trennwand (6), direkt gegenüber dem Paar Naseneinsätzen (5) angeordnete Strömungseinengungskanäle (35) sind.

**6.** Hilfsgerät zur Beatmung nach Anspruch 1, wobei das zweite Kompartiment (2) ein verformbares Wandreservoir ausbildet, das ein vollständig aufgeblähtes Innenvolumen für das Gas von etwa 0,5 bis 5 ml umfasst.

**7.** Hilfsgerät zur Beatmung nach Anspruch 1, wobei die Nasenkanülenanordnung keinen zum Erfassen eines Beginns einer Patienteneinatmung ausgelegten Sensor umfasst.

**8.** Hilfsgerät zur Beatmung nach Anspruch 1, wobei die Naseneinsätze (5) an einem Ende ein äußeres Kissenelement (8) beinhalten.

**9.** Hilfsgerät zur Beatmung nach Anspruch 8, wobei das Kissenelement (8) aus Silicon besteht.

**10.** Hilfsgerät zur Beatmung nach Anspruch 1, das weiter zwischen dem ersten Kompartiment (1), das eine Innenkammer (7) definiert, und einer äußeren Atmosphäre eine oder mehrere Öffnungen (13) umfasst.

**Revendications**

**1.** Appareil d'assistance respiratoire comprenant :

a) une source de gaz contenant du NO, et
b) un ensemble de canule nasale (10) en communication fluidique avec ladite source de gaz contenant du NO, l'ensemble de canule nasale (10) étant adapté à délivrer des gaz à un patient,

dans lequel l'ensemble de canule nasale (10) comprend :

A) un corps creux (4) configuré pour pouvoir agir comme un conduit de gaz ou un collecteur de gaz et comprenant une chambre interne (7) définissant un premier compartiment (1),
B) le premier compartiment (1) et un second compartiment (2) séparés par une paroi de séparation (6),
C) une paire de branches nasales (5) en communication fluidique avec le premier compartiment (1),
D) le premier compartiment (1) comprenant une première admission (11) formant une entrée de gaz latérale en communication fluidique avec un conduit de transport de gaz et configurée pour conduire un premier gaz dans ledit premier compartiment (1),
E) le second compartiment (2) ayant un volume interne entièrement gonflé pour un gaz, le second compartiment (2) comprenant,

une seconde admission (12) configurée pour conduire un second gaz dans ledit second compartiment (2), ledit second gaz étant un gaz contenant du NO provenant de la source de gaz contenant du NO, et une paroi déformable (14) formant une partie de la frontière entre le second compartiment (2) et l'atmosphère de la pièce,
et dans lequel la paroi de séparation (6) comprend au moins deux canaux de restriction d'écoulement (35).

**2.** Appareil d'assistance respiratoire selon la revendication 1, dans lequel ledit corps creux (4) et ladite paire de branches nasales (5) sont moulés intégralement en une matière plastique souple.

**3.** Appareil d'assistance respiratoire selon la revendication 1, dans lequel les branches (5) sont détachables dudit corps creux (4) et sont choisis parmi des branches de tailles différentes convenant à des narines de patients de tailles différentes.

**4.** Appareil d'assistance respiratoire selon la revendication 1, dans lequel les au moins deux canaux de restriction d'écoulement (35) relient le premier compartiment (1) et le second compartiment (2) et sont à bords arrondis à l'entrée du second compartiment (2) et ont une ouverture réentrante à l'entrée du premier compartiment (1).

**5.** Appareil d'assistance respiratoire selon la revendication 1, dans lequel les au moins deux canaux de restriction d'écoulement (35) sont deux canaux de restriction d'écoulement (35) agencés dans la paroi de séparation (6), directement opposés aux deux branches nasales (5).

**6.** Appareil d'assistance respiratoire selon la revendication 1, dans lequel le second compartiment (2) forme un réservoir à paroi déformable comprenant un volume interne entièrement gonflé pour le gaz d'environ 0,5 à 5 ml.

7. Appareil d'assistance respiratoire selon la revendication 1, dans lequel l'ensemble de canule nasale ne comprend pas de capteur configuré pour détecter un début d'inspiration du patient.

8. Appareil d'assistance respiratoire selon la revendication 1, dans lequel les branches nasales (5) comprennent un élément coussinet externe (8) à une extrémité.

9. Appareil d'assistance respiratoire selon la revendication 8, dans lequel ledit élément coussinet (8) est constitué de silicone.

10. Appareil d'assistance respiratoire selon la revendication 1, comprenant en outre un ou plusieurs orifices (13) entre le premier compartiment (1) définissant une chambre interne (7) et une atmosphère externe.

FIG 1

FIG 2

FIG 3

FIG 4

Inhalation w/
supplemental O₂:

Inhalation through patient's nares
Oxygen flow
NO-containing gas flow
Air entrained from room atmosphere

FIG 5A

Exhalation w/
supplemental O₂:

Exhalation from patient's nares
Oxygen flow
NO-containing gas flow

FIG 5B

Inhalation w/out
supplemental O₂:

| | Inhalation through patient's nares |
| | NO-containing gas flow |
| | Air entrained from room atmosphere |

FIG 5C

Exhalation w/out
supplemental $O_2$:

Exhalation from patient's nares

NO-containing gas flow

FIG 5D

FIG 6

FIG 7

FIG 8

FIG 9

FIG 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1516639 A
- US 5558083 A
- US 5873359 A
- US 5732693 A
- US 6051241 A
- US 6089229 A
- US 6142147 A
- US 6581599 B
- US 4535767 A, Tiep
- WO 2010076711 A

**Non-patent literature cited in the description**

- **DUMONT ; TIEP.** Using a reservoir nasal cannula in acute care. *Crit Care Nurse,* 2002, vol. 22, 41-46
- **FOX ; MCDONALD.** Introduction to Fluid Mechanics. John Wiley & Sons, 1998
- **SHAUGHNESSY ; KATZ ; SCHAFFER.** Introduction to Fluid Mechanics. Oxford University Press, 2005